# EUROPÄISCHE PATENTSCHRIFT

(11) **EP 1 795 170 B1**
(45) Veröffentlichungstag und Bekanntmachung des Hinweises auf die Patenterteilung: **21.01.2009**
(21) Anmeldenummer: 06012650.5
(22) Anmeldetag: 20.06.2006
(51) Int. Cl.: A61J 1/00, A61M 5/14

(54) **Vorrichtung zur Verarbeitung enteraler Ernährung**
Enteral nutrition administration device
Dispositif d'administration pour l'alimentation entérale

(30) Priorität: 06.12.2005 EP 05026568
(43) Veröffentlichungstag der Anmeldung: 13.06.2007
(73) Patentinhaber: Fritzmeier, Hildegard, 91710 Gunzenhausen (DE); Gemü GmbH, 6343 Rotkreuz (CH)
(72) Erfinder: Fritzmeier, Hildegard, 91710 Gunzenhausen (DE); Rominger, Lars, Dipl. Ing., 6313 Edlibach (CH); Brem, William, 8908 Hedingen (CH)
(74) Vertreter: Zech, Stefan Markus

(56) Entgegenhaltungen:
- EP-A- 0 355 795
- WO-A-85/05041
- DE-A1- 2 907 479
- DE-A1- 3 700 502
- DE-C1- 4 235 720
- US-A- 4 634 434
- US-A- 4 713 064
- US-A- 5 865 797

## Beschreibung

Die Erfindung betrifft eine Vorrichtung zur Verabreichung enteraler Ernährung umfassend ein erstes Reservoir sowie ein zweites Reservoir, die jeweils über eine Absperrverteilervorrichtung mit einem zum Patienten führenden Anschluss in Verbindung stehen, wobei das erste Reservoir zur Befüllung mit enteraler Ernährung und das zweite Reservoir zur Befüllung mit Flüssigkeit, insbesondere Tee oder Wasser vorgesehen ist, wobei die Absperrvorrichtung mit mehreren Funktionsstellungen ausgebildet ist, die zumindest ein Herstellen und Unterbrechen einer Strömungsverbindung zwischen dem ersten Reservoir und/oder dem zweiten Reservoir und dem Anschluss umfassen nach dem Oberbegriff vom Patentanspruch 1.

Eine derartige Vorrichtung zum Verabreichen enteraler Ernährung ist bereits aus der EP 1 129 682 A2 bekannt.

Aus der DE 29 07 479 A1 ist eine Zweikammerinfusionsflasche mit Doppelkorken und Verbindungskanal bekannt, welche erlaubt, Flüssigkeiten aus zwei getrennte Kammern mittels eines Infusionsgerätes vor Verabreichung zu vermischen. Hierbei muss das Infusionsgerät mittels Einstechdorn in die Infusionsflasche eingeführt werden.

Die US 5,865,797 beschreibt ein Zweikammerzuführungssystem, in welchem Flüssigkeiten aus zwei räumlich getrennten Kammern über getrennte schlauchartige Zuleitungen in eine Tropfkontrolleinheit zur Vermischung geleitet werden, wobei zwei Rollklemmen den Flüssigkeitszustrom regulieren.

Fernerhin ist eine Vorrichtung zur Vermischung von zu verabreichenden Flüssigkeiten aus der DE 42 35 720 C1 bekannt, in welcher ein Behältersystem mit zwei getrennten Flüssigkeitskammern die zu verabreichenden Flüssigkeiten enthält. Die Vermischung findet wiederum in einer separaten Entnahmevorrichtung statt, welche über Einstechdorne mit dem Behältersystem verbunden werden muss.

Generell zeichnen sich obige, dem Stand der Technik vorbekannte Vorrichtungen dadurch aus, dass die Aufbewahrung, Mischung und Dosierung mit Hilfe separater, zu verbindender Teilvorrichtungen ausgeführt werden. Dies beeinträchtigt sowohl die Handhabbarkeit als auch die Reproduzierbarkeit der Mischung und Dosierung.

Die Aufgabe der vorliegenden Erfindung besteht deshalb darin, eine derartige Vorrichtung in ihrer Handhabbarkeit noch zu verbessern und den Betrieb einer solchen Vorrichtung reproduzierbarer zu gestalten.

Diese Aufgabe wir mit einer Vorrichtung nach den Merkmalen des Anspruchs 1 gelöst. Vorteilhafte Weiterbildungen sind in den Unteransprüchen angegeben.

Ein Kerngedanke der vorliegenden Erfindung besteht darin, erstes Reservoir, zweites Reservoir und Absperrverteilervorrichtung als zumindest im Betriebszustand miteinander starr verbundene Einheit auzubilden. Ein weiterer Kerngedanke liegt ferner darin, dass die Absperrverteilervorrichtung Mittel zur Dosierung des an den Anschluss überführten Fluidstroms umfasst.

Im Gegensatz zur vorbekannten Vorrichtung ist die erfindungsgemäße Vorrichtung wesentlich kompakter aufgebaut und darüber hinaus auch in der Handhabbarkeit entscheidend verbessert. Der an der Absperrverteilervorrichtung anstehende Fluiddruck der enteralen Ernährung bzw. der Flüssigkeit ist reproduzierbarer, da erfindungsgemäß erstes Reservoir und zweites Reservoir im Betriebszustand mit der Absperrverteilervorrichtung starr verbunden sind. Bei der vorbekannten Vorrichtung hängt dies hingegen von der Aufhängehöhe des jeweiligen Reservoirs ab. Möglicherweise ist dies ein Grund, weshalb sich das vorbekannte System bislang nicht durchsetzen konnte.

Ferner umfasst die Absperrverteilervorrichtung Mittel zur Dosierung des an den Anschluss überführten Fluidstroms. Damit übernimmt die Absperrverteilervorrichtung nicht nur die Funktion des Herstellens bzw. Unterbrechens einer Strömungsverbindung zwischen dem ersten Reservoir und/oder dem zweiten Reservoir und dem Anschluss, sondern ist gleichzeitig in der Lage, diesen Fluidstrom, der aus Bezug von Substanz aus dem ersten Reservoir und/oder zweiten Reservoir herrührt, zu dosieren. Eine externe Dosiervorrichtung, wie bei vorbekannten Systemen vorgesehen, kann in der erfindungsgemäßen Vorrichtung daher entfallen.

In der ersten alternativen Ausgestaltung sind erstes Reservoir und/oder zweites Reservoir und Absperrverteilervorrichtung einstückig zusammenhängend ausgebildet. Es ist aber auch möglich erstes Reservoir und/oder zweites Reservoir von der Absperrverteilervorrichtung, beispielsweise über einen Schraubanschluss oder über einen Einschnappanschluss lösbar auszubilden. Ein Einschnappanschluss wird wegen seiner verbesserten Handhabbarkeit bevorzugt.

In einer besonders vorteilhaften Ausgestaltung der vorliegenden Erfindung sind erstes Reservoir und zweites Reservoir durch ein gemeinsames Flächenstück, das eine Trennwand zwischen erstem Reservoir und zweitem Reservoir ausbildet, getrennt. Erstes Reservoir und zweites Reservoir grenzen damit beide unmittelbar aneinander an, was die Kompaktheit der gesamten Vorrichtung noch erhöht.

Nach einem bevorzugten Aspekt der vorliegenden Erfindung bilden erstes Reservoir und zweites Reservoir ein zusammenhängendes Behältnis mit einer gemeinsamen Außenwand aus. Hierdurch wird die kompakte Bauform noch begünstigt. Es entsteht ein Gesamtbehältnis mit zwei getrennten Kompartimenten, die jeweils erstes und zweites Reservoir ausbilden.

Gemäß einer alternativen, ebenfalls bevorzugten Lösung sind erstes Reservoir und zweites Reservoir jeweils als separate Einheiten ausgebildet. Vorzugsweise werden sie aneinander über Verkopplungsmittel lösbar befestigt, sodass eine im Betriebszustand starr zusammenhängende Einheit gebildet wird. Gleichzeitig sind die Reservoire aber auch voneinander lösbar, etwa um sie einzeln auszutauschen.

In einer weiter bevorzugten Ausgestaltung ist die Absperrverteilervorrichtung dazu ausgebildet, ein gewünschtes Mischungsverhältnis zwischen Entnahme von enteraler Ernährung aus dem Reservoir und Entnahme von Flüssigkeit aus dem zweiten Reservoir einzustellen. In dieser Ausgestaltung sind beliebige Mischungen aus enteraler Ernährung und Flüssigkeit, beispielsweise Tee möglich, insbesondere um enterale Ernährung bei Ernährungsbeginn zu verdünnen. Auch überkalorische Nährungen können stufenlos herunter verdünnt werden.

Gleichzeitig ist es möglich, etwa zum Spülen der Sonde oder um nicht selten auftretende Verstopfungen in dem bei der Überleitung an den Patienten verwendeten Sonden zu vermeiden, das Mischungsverhältnis kurzfristig auf wesentlich höhere Flüssigkeitsgabe bzw. Teegabe oder auch gänzlich auf Flüssigkeit bzw. Tee umzustellen.

Gemäß einer ersten alternativen Ausgestaltung umfasst die Absperrverteilervorrichtung zwei den ersten Reservoir und dem zweiten Reservoir jeweils zugeordnete, getrennte Dosierventile. Hiermit lässt sich die Entnahme aus dem ersten Reservoir getrennt von der Entnahme aus dem zweiten Reservoir über jeweils einzelne Ventile einstellen.

Gemäß einer zweiten alternativen Ausführungsform umfasst die Absperrverteilervorrichtung ein gemeinsames dem ersten Reservoir und dem zweiten Reservoir zugeordnetes Misch/Dosierventile. In dieser Ausgestaltung lassen sich Mischungsverhältnis und Fluidstrom der insgesamt entnommen Substanz mittels eines einzigen Misch-/Dosierventils einstellen. Mit einer einzigen Betätigungshandhabe lassen sich also sowohl Mischungsverhältnis als auch der Volumenstrom der entnommen Substanz steuern.

In einer konkreten Ausgestaltung umfasst die Absperrverteilervorrichtung in beiden vorgenannten Alternativen ein mit dem ersten Reservoir und/oder dem zweiten Reservoir einstückig verbundenes oder starr verbindbares Außenelement mit mindestens zwei Einlassen und mindestens einem Auslass. Innerhalb dieses Außenelements sind gemäß der ersten Alternative die zwei Dosierventile oder gemäß der zweiten Alternative das einzige Misch-/Dosierventil ausgebildet bzw. gelagert.

Gemäß der ersten Alternative mit zwei unabhängigen Dosierventilen können diese in einer konkreten Weiterbildung jeweils ein gegenüber dem Außenelement drehbewegliches Innenelement umfassen. Gemäß der anderen alternativen Ausgestaltung mit lediglich einem einzigen Misch-/Dosierventil umfasst dieses ein über den Außenelement sowohl drehbeweglich als auch translatorisch verschiebbar gelagertes Innenelement.

In einer weiter konkretisierten Ausgestaltung umfasst das Innenelement der Dosierventile oder das Innenelement des Misch-/Dosierventils mindestens eine um einen vorgegebenen Winkelbereich, vorzugsweise von etwa 140 °, umlaufende, in ihrer Tiefe stetig zunehmende Dosiernut. Mit rotatorischer Verstellung des Innenelements kann über den für den Fluidstrom wirksamen Querschnitt der Nut die Dosierung der Entnahme aus erstem Reservoir und/oder zweitem Reservoir eingestellt werden.

In der Ausgestaltung, in der die Absperrverteilervorrichtung lediglich ein einziges Misch-/Dosierventil umfasst, kann dessen Innenelement zwei parallel zueinander verlaufende, in der Zunahme ihrer Tiefe jedoch entgegengesetzt ausgerichtete Dosiernuten, die jeweils einen der beiden Reservoire zugeordnet sind, aufweisen.

In einer bevorzugten Weiterbildung der vorgenannten Ausgestaltung ist zwischen den beiden Dosiernuten eine Mischnut angeordnet und beide Dosiernuten stehen mit der Mischnut in Fluidverbindung, wobei in der Mischnut eine Vermischung der aus den beiden Dosiernuten austretenden Fluidströme stattfindet.

Ebenfalls in der Ausgestaltung, in der die Absperrverteilervorrichtung lediglich ein einziges Misch-/Dosierventil aufweist, kann dessen Innenelement an seinem Außenumfang einen Abschnitt aufweisen, der einen Dosierkonus ausbildet, um so durch translatorische Verschiebung des Innenelements relativ zum Außenelement eine Dosierung des an den Anschluss überführten Fluidstroms zu ermöglichen. Konkret kann durch Verschiebung des Dosierkonus der für den Fluidstrom wirksame offene Querschnitt vergrößert bzw. verkleinert werden.

Damit die bereits gemischte Substanz aus der Mischnut an den Dosierkonus geführt werden kann, ist in einer weiter bevorzugten Ausgestaltung eine Überführungsnut ausgebildet, um Fluid aus der Mischnut an den Dosierkonus zu überführen.

Unabhängig von der Ausbildung der Absperrverteilervorrichtung mit mehreren einzelnen Dosierventilen oder einem einzigen Misch-/Dosierventil können die Innenelemente der Dosierventile bzw. das Innenelement des Misch-/Dosierventils mittels einer Verrastung im Außenelement drehbeweglich fixiert werden. Konkret können die Innenelemente hierfür Rastnasen aufweisen, die in einen korrespondierenden Rücksprung bzw. in eine korrespondierende Nut oder Ausnehmung eingreifen.

In einer bevorzugten Ausgestaltung sind erstes Reservoir und zweites Reservoir jeweils mit einem Fassungsvolumen von 600 ml bis 1500 ml, bevorzugt von jeweils 1000 ml ausgebildet.

In einer weiteren Ausgestaltung umfassen erstes Reservoir und/oder zweites Reservoir an ihrer der Absperrverteilervorrichtung abgewandten Seite jeweils eine, vorzugsweise mit Deckel verschließbare Befüllöffnung, zur Nachfüllung von enteraler Ernährung bzw. von Flüssigkeit.

In einer besonders bevorzugten Ausgestaltung entspricht die Befüllöffnung zumindest im wesentlich dem Innenquerschnitt des zugeordneten Reservoirs. Durch eine derart weite Befüllöffnung lässt sich das jeweilige Reservoir gut reinigen, da es auch mit mechanischem Reinigungsgerät von außen gut zugänglich ist. Darüber hinaus wird die Befüllung in entscheidendem Maße vereinfacht. Enterale Ernährung kann besonders preiswert primär aus Schlauchbeuteln aus Aluminiumverbundfolie nachgefüllt werden. In Schlauchbeuteln aus Aluminiumverbundfolie besteht optimaler Schutz vor Licht und Sauerstoff und damit lange Haltbarkeit, wobei ein Öffnen des Schlauchbeutels erst unmittelbar vor Verabreichung an den Patienten trotz der erfindungsgemäßen Vorrichtung notwendig ist. Das Umfüllen in das zugeordnete Reservoir der erfindungsgemäßen Vorrichtung gelingt hierbei ohne Hilfsmittel. Auch eine Nachbefüllung aus Glasflaschen ist ohne weiteres möglich und gelingt ebenfalls ohne Hilfsmittel.

Bevorzugtermaßen sind Außenelement und Innenelement so ausgebildet und aufeinander abgestimmt, dass für das Einstellen des Mischungsverhältnisses zwischen Entnahme aus dem ersten Reservoir und Entnahme aus dem zweiten Reservoir und/oder für das Einstellen des Fluidstroms/der Abgabemenge an den Anschluss die Absperrverteilervorrichtung eine vorbestimmte Anzahl definierter Rastpositionen umfasst, um das Mischungsverhältnis bzw. die Abgabemenge in diskreten Schritten einstellen zu können. Hierdurch wird eine reproduzierbare Dosierung erleichtert.

Alternativ ist es aber auch möglich Außenelement und Innenelement so auszubilden und aufeinander abzustimmen, dass eine kontinuierliche Einstellung des Mischungsverhältnisses zwischen Entnahme aus dem ersten Reservoir und Entnahme aus dem zweiten Reservoir und/oder für das Einstellen des Fluidstroms/der Abgabemenge an den Anschluss vorgesehen ist.

Die Erfindung wird nachstehend auch hinsichtlich weiterer Merkmale und Vorteile anhand der Beschreibung von Ausführungsbeispielen und unter Bezugnahme auf die nachstehenden Zeichnungen näher erläutert.

Hierbei zeigen:
- Fig. 1:: eine schematische Darstellung einer möglichen Ausführungsform der erfindungsgemäßen Vorrichtung in perspektivischer Ansicht.
- Fig. 2:: eine Explosionsdarstellung der anhand von Fig. 1 veranschaulichten Ausführungsform, ebenfalls in perspektivischer Ansicht
- Fig. 3: eine Schnittansicht der Vorrichtung aus Fig. 1 entlang der Linie III
- Fig. 4: eine weitere, bevorzugte Ausführungsform der erfindungsgemäßen Vorrichtung in perspektivischer Ansicht
- Fig. 5: die Ausführungsform nach Fig. 4 aus einer zur Ansicht nach Fig. 4 abweichenden perspektivischen Ansicht
- Fig. 6: erstes bzw. zweites Reservoir gemäß der Ausführungsform nach Fig. 5 bzw. Fig. 6
- Fig. 7: das bei der Ausführungsform nach Fig. 5 bzw. Fig. 6 zum Einsatz gelangende Innenelement
- Fig. 8: das bei der Ausführungsform nach Fig. 5 bzw. Fig. 6 zum Einsatz gelangende Außenelement in einer ersten perspektivischen Ansicht
- Fig. 9: das bei der Ausführungsform nach Fig. 5 bzw. Fig. 6 zum Einsatz gelangende Außenelement in einer zweiten perspektivischen Ansicht
- Fig. 10: ein bevorzugter Deckel für das erste bzw. zweite Reservoir bei der Ausführungsform nach den Fig. 4 bis 9
- Fig. 11: eine weitere besonders bevorzugte Ausführungsform der erfindungsgemäßen Vorrichtung in perspektivischer Ansicht
- Fig. 12: eine schematische Prinzipansicht zur Veranschaulichung des Zusammenwirkens von Innenelement und Außenelement bei der Ausführungsform nach Fig. 11 in einer ersten Ansicht
- Fig. 13: eine schematische Prinzipansicht zur Veranschaulichung des Zusammenwirkens von Innenelement und Außenelement bei der Ausführungsform nach Fig. 11 in einer zweiten Ansicht
- Fig. 14: eine schematische Prinzipansicht zur Veranschaulichung des Zusammenwirkens von Innenelement und Außenelement bei der Ausführungsform nach Fig. 11 in einer dritten Ansicht
- Fig. 15: eine perspektivische Ansicht der Absperrverteilervorrichtung umfassend Innenelement und Außenelement bei der besonders bevorzugten Ausführungsform nach Fig. 12

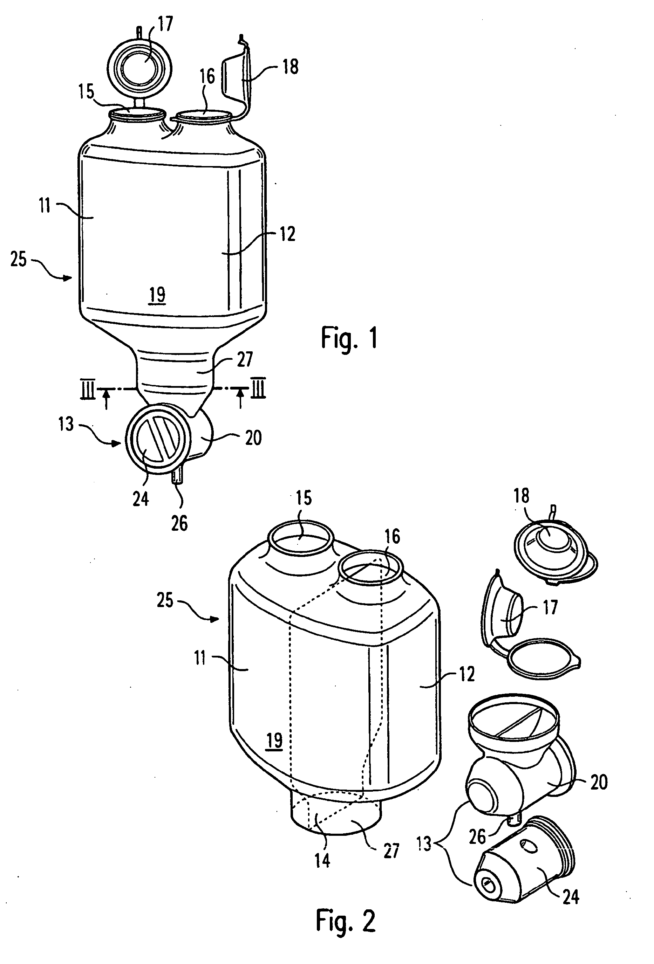

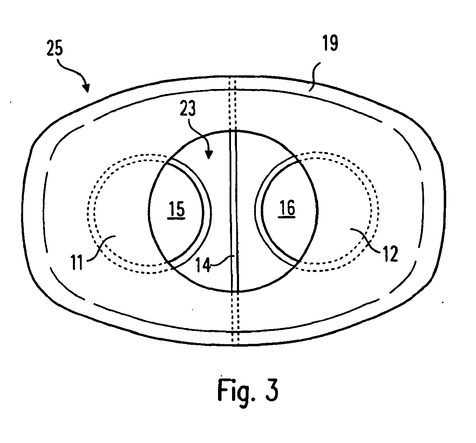

Anhand der Fig. 1 bis 3 ist eine bevorzugte Ausführungsform der erfindungsgemäßen Vorrichtung umfassend ein Behältnis 25 mit einem Überführungsabschnitt 27 sowie einer am Überführungsabschnitt 27 starr angeschlossenen Absperrverteilervorrichtung 13, veranschaulicht. Das Behältnis 25 weist eine umlaufende Außenwand 19 auf und ist über eine Trennwand 14 in zwei - hier gleich große - Kompartimente unterteilt, die ein erstes Reservoir 11 und ein zweites Reservoir 12 ausbilden.

Erstes Reservoir 11 und zweites Reservoir 12 sind jeweils über Befüllöffnungen 15, 16 mit enteraler Ernährung bzw. Flüssigkeit, insbesondere Wasser oder Tee befüllbar. Die Befüllöffnungen 15, 16 lassen sich über Deckel 17, 18 verschließen.

Die Absperrverteilervorrichtung 13 kann mit dem Behältnis 25 dauerhaft, insbesondere über Kleben, Schweißen oder einstückige Ausbildung im Formprozess dauerhaft verbunden sein. Die Absperrverteilervorrichtung 13 umfasst ein Außenelement 20 sowie ein gegenüber dem Außenelement 20 drehbeweglich und translatorisch verschiebbar gelagertes Innenelement 24. Das Außenelement 20 umfasst dem Innenelement 24 zugewandt mindestens zwei Einlässe sowie mindestens einen Auslass.

Durch Drehen des Innenelements 24 im Außenelement 20 lassen sich die gewünschten Funktionsstellungen hinsichtlich Herstellen und Unterbrechen einer Strömungsverbindung zwischen dem ersten Reservoir 11 oder dem zweiten Reservoir 12 und einem Anschluss 26 bzw. Mischungsverhältnisse zwischen Entnahme von enteraler Ernährung aus dem ersten Reservoir 11 und Entnahme von Flüssigkeit aus dem zweiten Reservoir 12 einstellen. Durch translatorisches Verschieben des Innenelements 24 im Außenelement 20 lässt sich die an den Anschluss 26 überführte Abgabemenge dosieren. An den Anschluss 26 können an sich bekannte Überleitungssysteme angeschlossen werden oder bereits angeschlossen sein.

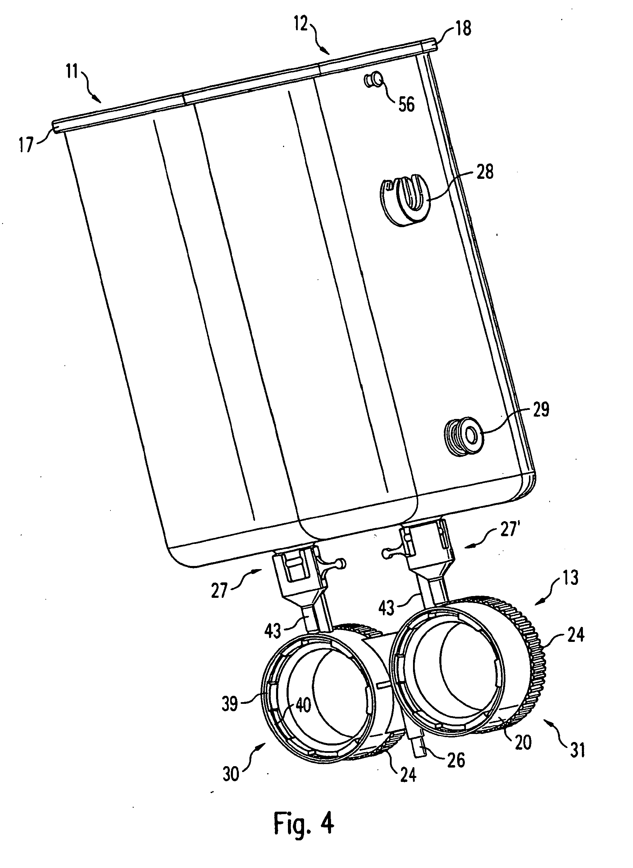

In den Fig. 4 bis 6 ist eine weitere, bevorzugte Ausführungsform der erfindungsgemäßen Vorrichtung dargestellt. Die vorliegende Ausführungsform unterscheidet sich von der Ausführungsform nach den Fig. 1 bis 3 in mehreren Gesichtspunkten, insbesondere darin, dass erstes Reservoir 11 und zweites Reservoir 12 als separate, voneinander lösbare Einheiten ausgebildet sind. Zu diesem Zweck weisen sowohl erstes Reservoir 11 als auch zweites Reservoir 12 Verkopplungsmittel 28, 29 auf. Diese sind seitlich an der Außenwand der Reservoire 11, 12 vorgesehen, und zwar in der Weise, dass die Reservoire 11, 12 jeweils einschließlich ihrer Verkopplungsmittel 28, 29 identisch ausgebildet sein können und miteinander - hier durch Relativverschiebung und einer anschließenden Schwenkbewegung gegeneinander - verkoppelt werden können. Theoretisch wäre es denkbar, auch noch ein drittes oder weitere Reservoire anzukoppeln, wobei in diesem Fall die Absperrverteilervorrichtung 13 entsprechend zu modifizieren wäre. An ihren distalen Enden können erstes Reservoir 11 und zweites Reservoir 12 noch Aufhängeinrichtungen 56 aufweisen, um die Gesamtvorrichtung beispielsweise über einen Federbügel, ein Gummiband o. ä. in Gebrauchslage aufzuhängen.

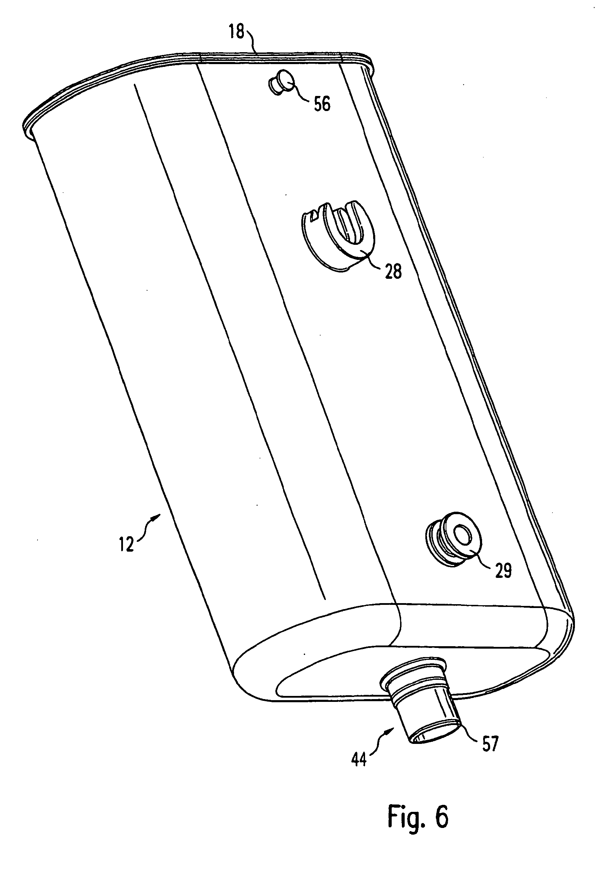

Im vorliegenden Fall umfassen Überführungsabschnitte 27, 27' zwischen erstem Reservoir 11 bzw. zweitem Reservoir 12 und Absperrverteilervorrichtung 13 zwei der Absperrverteilervorrichtung zugeordnete Einlassstutzen 43 sowie einen am Reservoir 11 bzw. 12 jeweils angeformten Auslassstutzen 44. Auslassstutzen 44 und Einlassstutzen 43 sind so aufeinander abgestimmt, dass jeweils ein Einschnappanschluss 41 definiert wird. Dabei wird durch den Einschnappanschluss 41 der Einlassstutzen 43 so im Auslassstutzen gehalten, dass eine stirnseitige Radfläche 57 des Einlassstutzen 43 gegen den Auslassstutzen 44 abdichtet (vgl. Fig. 6).

Die beiden Einlassstutzen 43 sind als einstückige Bestandteile des Außenelements 20 der Absperrverteilervorrichtung 13 und mit ebenfalls einstückig angeformten Federzungen 58 bis 60 versehen, zur Ausbildung des bereits erwähnten Einschnappanschlusses 41. Mindestens eine der Federzungen 58 bis 60 kann mit einer Handhabe 61 versehen sein, um den Einschnappanschluss 41 leichter lösen zu können. Die Absperrverteilervorrichtung 13 der Ausführungsform nach den Fig. 4 bis 10 umfasst ein Außenelement 20, welches ein erstes Ringelement 49 sowie ein zweites Ringelement 50 ausbildet, sowie zwei separat verstellbare Innenelemente 24, die von im Wesentlichen zylindrischer Grundform in entsprechenden durch erstes und zweites Ringelement 49, 50 definierten Ausnehmungen des Außenelements 20 jeweils drehbeweglich gelagert sind. Die Innenelemente 24 sind in den Ringelementen 49, 50 im Außenelement 20 eingeschnappt. Hierfür weist das Außenelement 20 in der jeweiligen Ausnehmung bzw. im jeweiligen Ringelement 49, 50 einen Rücksprung 40 auf. Die Innenelemente sind zum Eingriff in die Rücksprünge 40 im Außenelement 20 mit einer Mehrzahl von Rastnasen 39 ausgebildet.

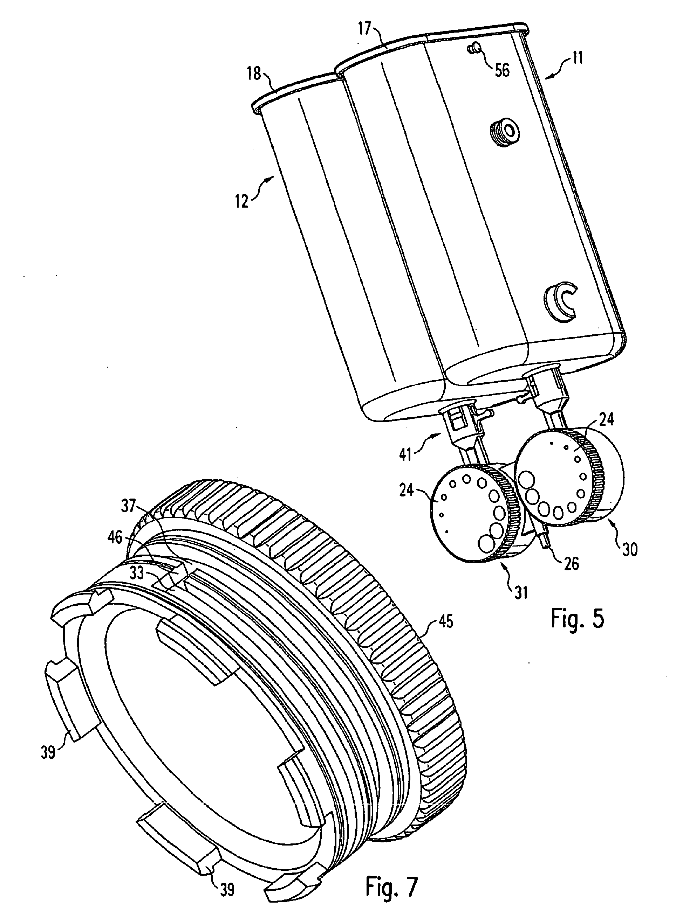

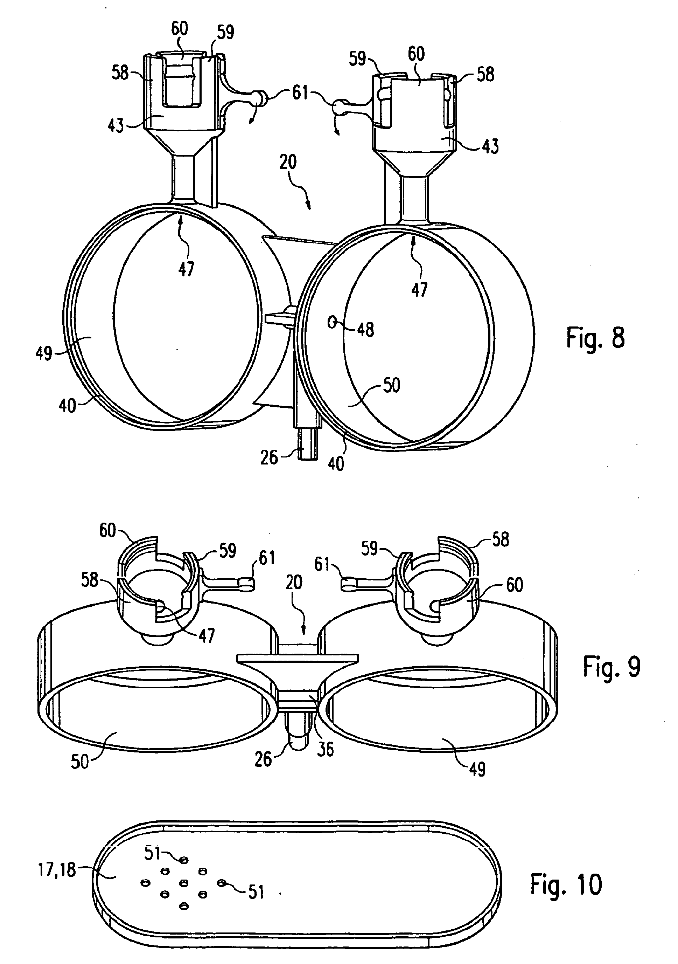

Der Aufbau der Innenelemente 24 wird aus Fig. 7, in der eines der Innenelemente 24 gezeigt ist noch klarer verständlich. Entsprechend wird aus den Ansichten nach den Fig. 8 und 9 der Aufbau des Außenelementes 20 noch klarer verständlich. Das Innenelement 24 weist an seinem einen Ende die bereits erwähnten Rastnasen 39 und an seinem gegenüberliegenden Ende ein auch im eingesetzten Zustand über das Außenelement 20 vorstehendes Betätigungsrad 45 auf. Zwischen Rastnasen 39 und Betätigungsrad 45 laufen am Außenumfang des Innenelements 24 zueinander parallel eine Mischnut 37 sowie eine Dosiernut 33. Dabei ist die Mischnut 37 dem Betätigungsrad zugewandt und die Dosiernut 33 zwischen der Mischnut 37 und den Rastnasen 39 angeordnet. Die Nuttiefe der Dosiernut 33 ändert sich über den Umfang des Innenelements 24 stetig derart, dass die Dosiernut 33 an einer Überführungsnut 46 zur Mischnut 37 hin die größte Tiefe erreicht hat. Die Dosiernut 33 kann sich - stetig zunehmend - beispielsweise über einen Winkelbereich von 270° erstrecken. Die Innenelemente 24 können beispielsweise aus HD-PE oder einem anderen geeigneten Material gebildet sein.

Im Außenelement 20 (vgl. Fig. 8 und 9) sind zentral in den Einlassstutzen 43 jeweils eine Einlassbohrung 47 angeordnet, über die Substanz aus dem ersten Reservoir 11 bzw. zweiten Reservoir 12 in die Dosiernut 33 des jeweils zugeordneten Innenelements 24 geführt werden kann. Das Innenelement 24 ist deshalb so im Außenelement 20 gelagert, dass die Einlassbohrung 47 direkt auf die Dosiernut 33 ausgerichtet ist.

Eine Auslassbohrung 48 ist zur Mischnut 37 fluchtend ausgerichtet, derart, dass in die Dosiernut 33 einströmende Substanz über die Überführungsnut 46 in die Mischnut 37 und von dort in die Auslassbohrung 48 eintreten kann. Die Auslassbohrung 48 beider Innenelemente 24 sind miteinander verbunden, vorzugsweise als eine gemeinsam zusammenhängende Bohrung ausgebildet und definieren eine Mischstelle 36 von der ausgehend die ggf. vermischte Substanz an einen Anschluss 26 geführt wird. Das Außenelement 20 kann transparent ausgebildet sein, um den Durchfluss der Substanzen verfolgen zu können. Als geeignete Materialien kommen insbesondere Kunststoffe wie PC oder PS oder ähnliche Kunststoffe in Betracht. Jeweils ein Innenelement 24 definiert zusammen mit dem zugeordneten Ringelement 49, 50 des Außenelements 20 ein Dosierventil 30, 31, wodurch eine zunächst relativ unabhängige Einstellung der Menge und in Wechselwirkung mit dem jeweils anderen Dosierventil 31, 30 auch das Mischverhältnis einstellbar ist. Je nach radialer Stellung des einstückig mit dem Innenelement 24 zusammenhängenden Betätigungsrades erfolgt eine Reglung des Volumenstromes von 0 bis 100%.

Folgende Funktionen können mit der Vorrichtung bzw. mit dem System nach der Ausführungsform in den Fig. 4 bis 10 geregelt werden:
- Einstellung Medium A einzeln von 0 bis 100% Volumenstrom
- Einstellung Medium B einzeln von 0 bis 100% Volumenstrom
- Einstellung Medium A und B parallel von 0 bis 100%.

In Figur 10 ist schließlich noch ein Deckel 17 zur Überdeckung einer an der dem Einlassstutzen 43 jeweils abgewandten Seite des Reservoirs 11, 12 vorgesehenen Befüllöffnung 15, 16 veranschaulicht. Der Deckel 17 bzw. der Deckel 18 ist noch mit Belüftungslöchern 51 versehen, um einen ungehinderten Abfluss der jeweils im Reservoir 11, 12 aufgenommenen Substanz bei entsprechender Einstellung des Innenelements 24 zu gewährleisten.

Bei der Ausführungsform nach den Fig. 4 bis 10 kann die Dosierung der enteralen Ernährung einerseits und/oder der Flüssigkeit, insbesondere Tee oder Wasser andererseits relativ unabhängig durch die zwei getrennten durch die Innenelemente 24 definierten Dosierränder eingestellt werden, wobei gerade bei höheren Durchflussraten die Unabhängigkeit der Einstellung durch ein Aufeinandertreffen der beiden Ströme an der Mischstelle 36 zumindest teilweise relativiert sein könnte.

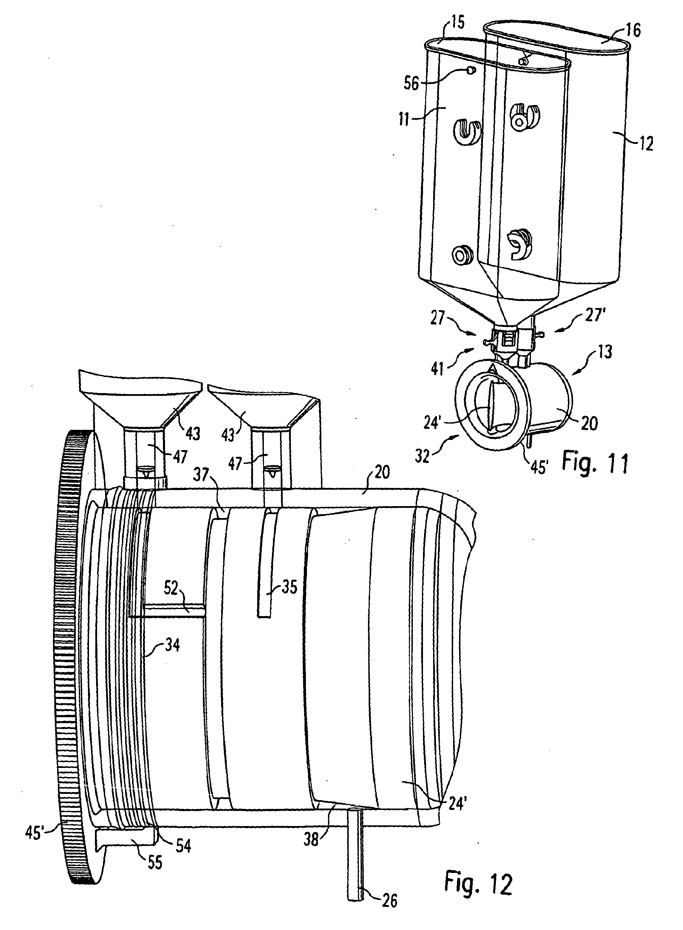

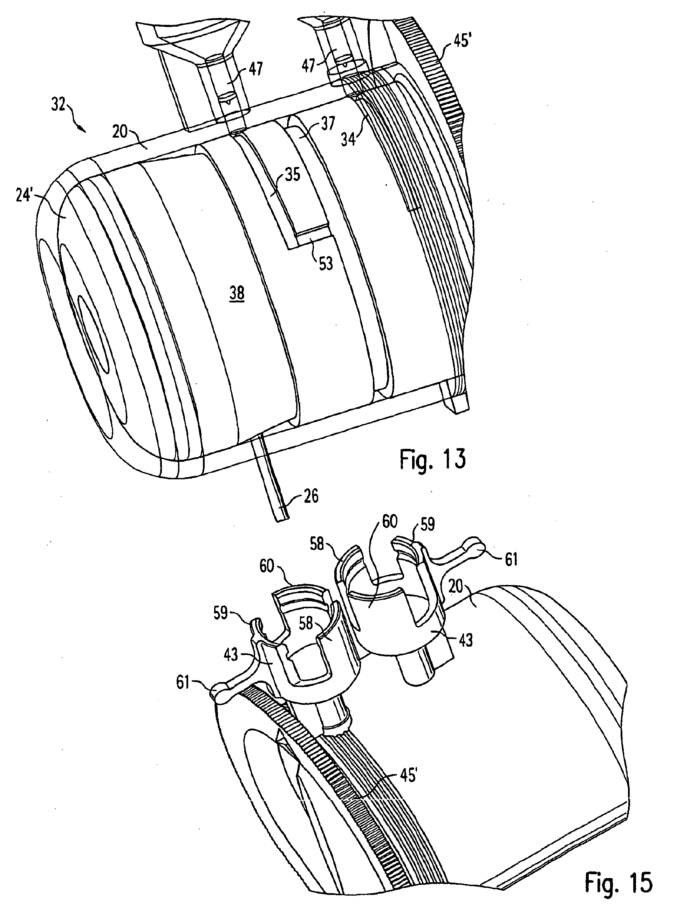

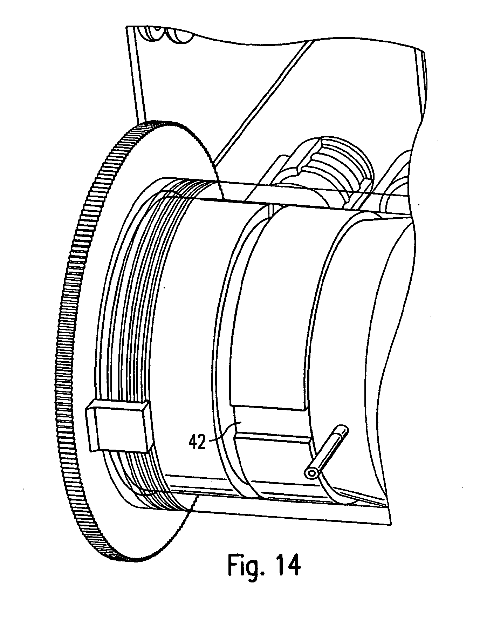

In den Fig. 11 bis 15 ist eine weitere, besonders bevorzugte Ausführungsform der vorliegenden Erfindung dargestellt. Bei der anhand der Fig. 11 bis 15 veranschaulichten Ausführungsform ist die Absperrverteilervorrichtung 13 mit nur einem Innenelement 24' ausgebildet, das heißt wie schon anhand der in den Fig. 1 bis 3 beschriebenen Ausführungsform wird hier der Fluidstrom beider Substanzen durch rotatorische bzw. translatorische Verstellung des einzigen Innenelements 24' eingestellt.

In Figur 11 ist zunächst der Gesamtaufbau der Vorrichtung skizzenhaft veranschaulicht. Erstes Reservoir 11 und zweites Reservoir 12 sind entsprechend erstem Reservoir 11 und zweitem Reservoir 12 nach der Ausführungsform gemäß Fig. 4 bis 10 aufgebaut, das heißt auch in der vorliegenden Ausführungsform weisen erstes Reservoir 11 und zweites Reservoir 12 jeweils einen Auslassstutzen 44 auf, der in einem Einlassstutzen 43 des Außenelements 20 aufgenommen ist, wobei Einlassstutzen 43 und Auslassstutzen 44 unter Ausbildung eines Einschnappanschlusses 41 miteinander zusammenwirken. Auch der Deckel 17 bzw. 18 kann bei dem Reservoir 11 bzw. Reservoir 12 nach den Fig. 11 bis 15 zum Einsatz gelangen.

Die beiden Einlassstutzen 43 des Außenelements 20 sind auf einer sich parallel zur Drehachse des Innenelementes 24' erstreckenden Linie auf dem Außenelement 20 angeordnet, so dass auch hier durch Einschnappen der miteinander verkoppelten Reservoire 11, 12 in die Einlassstutzen 43 eine insgesamt starre Gesamtanordnung geschaffen wird. Das Zusammenwirken des Innenelementes 24' mit dem Außenelement 20 wird nun anhand der Fig. 12 bis 14 noch detaillierter erläutert. Ausgehend vom Einlassstutzen 43 sind jeweils Einlassbohrungen 47 vorgesehen. Die dem Betätigungsrad 45' zugewandte Einlassbohrung 47 korrespondiert mit einer ersten Dosiernut 34. Die dem Betätigungsrad 45' abgewandten Einlassbohrung 47 korrespondiert mit einer zweiten Dosiernut 35. Beide Dosiernuten 34, 35 sind jeweils über eine Überführungsnut 52, 53 mit einer Mischnut 37 verbunden. Die Tiefen der Dosiernuten 34, 35 nehmen ausgehend von einem Startpunkt bis zur jeweiligen Überführungsnut 52, 53 kontinuierlich, jedoch gegenläufig zueinander zu, so dass je nach Winkelposition des Innenelements 24' der Fluidstrom aus dem ersten Reservoir 11 oder aus dem zweiten Reservoir 12 begünstigt ist, sich also das Mischungsverhältnis zwischen Entnahme aus erstem Reservoir 11 und zweitem Reservoir 12 auf diese Weise einstellen lässt.

Die entsprechend vordosierten Fluidströme gelangen über die Überführungsnut 52, 53 in eine gemeinsame Mischnut 37 und von dort über eine Überführungsnut 42 in einen durch einen Dosierkonus 38 und das Außenelement 20 gebildeten Raum. Je nach Positionierung des Innenelements 24' innerhalb des Außenelements 20 wird ein größerer oder kleinerer Zuflussquerschnitt einer Auslassöffnung 54, über die das gemischte Fluid in den Anschluss 26 einströmt, ermöglicht.

Die Einstellung der abgegebenen Gesamtmenge erfolgt - wie bereits erwähnt - durch translatorische Verschiebung des Innenelements 24'. Dies kann stufenlos oder durch Vorgabe bestimmter Rastpositionen ermöglicht werden. In der vorliegenden Ausführungsform sind eine Mehrzahl definierter Rastpositionen entsprechend unterschiedlicher Fluidströme, nämlich insgesamt vier Rastpositionen vorgegeben. Hierzu sind in der Außenfläche des Außenelements 20 mehrere Rastnuten 54 angeordnet, in die ein mit dem Innenelement 24' verbundenes, federndes Rastelement 55 eingreift.

Eine Anzeige des Mischverhältnisses kann in der Absperrverteilervorrichtung 13 konkret durch Ausbildung einer Skala am Innenelement 24' und/oder Außenelement 20 integriert werden. Weiterhin kann auch eine Anzeige des Volumenstroms in der Absperrverteilervorrichtung 13, konkret ebenfalls durch eine Skala im Innenelement 24 und/oder Außenelement 20, integriert werden.

Generell ist darauf hinzuweisen, dass der bei beiden Ausführungsformen sowohl nach den Fig. 4 bis 10 als auch den Fig. 11 bis 14 zum Einsatz gelangende Einschnappanschluss 41 sehr leicht, insbesondere auch nur einhändig, bedient werden kann.

Die erfindungsgemäße Vorrichtung ermöglicht die Gabe von enteraler Ernährung und von Flüssigkeit, insbesondere von Tee oder Wasser, nacheinander oder in beliebigem Mischungsverhältnis sowie in der gewünschten Dosierung. Über die Dosierung lassen sich die üblicherweise geforderten Tropfraten zwischen 25 ml/h bis maximal 300ml/h einstellen. Eine Rollenbremse oder Pumpe ist bei der erfindungsgemäßen Vorrichtung verzichtbar.

Die erfindungsgemäße Vorrichtung ermöglicht auch nach der Gabe der enteralen Ernährung auf Flüssigkeit, insbesondere Tee bzw. Wasser zur Flüssigkeitsgabe oder zum Spülen der Überleitungsmimik inklusive der Sonde umzustellen.

Das Umwechseln auf ein anderes System oder die Füllung des enteralen Systems mit Tee/Wasser nach Gabe der enteralen Ernährung ist bei der erfmdungsgemäßen Vorrichtung nicht notwendig. Das mindestens zwei Reservoirs 11, 12 umfassende Behältnis 25 kann preiswert mit enteraler Ernährung aus Flaschen oder Beuteln befüllt werden. Das Behältnis 25 sowie die daran befestigte oder befestigbare Absperrverteilervorrichtung 13 können ohne weiteres zehn Tage und länger für den gleichen Patienten verwendet werden. Am Ende der täglichen Anwendung erfolgt die Reinigung in einer Spülmaschine, vorzugsweise in einer medizinischen Spülmaschine (95°C).

In den Ausführungsformen nach den Fig. 4 bis 10 sowie Fig. 11 bis 15 ist das Innenelement 24 der Absperrverteilervorrichtung 13 vom Außenelement 20 lösbar bzw. zusätzlich die Absperrverteilervorrichtung 13 insgesamt vom Behältnis 25 lösbar, so dass es auch möglich ist, Innenelement 24 bzw. die gesamte Absperrverteilervorrichtung 13 nach einer Nutzungsdauer von einem Tag zu entfernen und zu entsorgen.

Die Kosten des Gesamtsystems können so, bei gleichzeitig bequemer Handhabung, niedrig gehalten werden.

Ein neues Innenelement 24 bzw. eine neue Absperrverteilervorrichtung 13 als Austauschteil kann ggf. mit anhängendem Überleitungssystem nach MPG sterilisiert und verpackt werden, wie dies bei herkömmlichen Überleitungsgeräten auch bislang der Fall ist.

### Bezugszeichenliste

- 11: erstes Reservoir
- 12: zweites Reservoir
- 13: Absperrverteilervorrichtung
- 14: Trennwand
- 15, 16: Befüllöffnung
- 17, 18: Deckel
- 19: Außenwand
- 20: Außenelement
- 24, 24': Innenelement
- 25: Behältnis
- 26: Anschluss
- 27, 27': Überführungsabschnitt
- 28: Verkopplungsmittel
- 29: Verkopplungsmittel
- 30, 31: Dosierventile
- 32: Misch-/Dosierventil
- 33: Dosiernut (Dosierventile)
- 34, 35: Dosiernut (Misch-/Dosierventil)
- 36: Mischstelle
- 37: Mischnut
- 38: Dosierkonus
- 39: Rastnasen
- 40: Rücksprünge
- 41: Einschnappanschluss
- 42: Überführungsnut
- 43: Einlassstutzen
- 44: Auslassstutzen
- 45, 45': Betätigungsrad
- 46: Überführungsnut
- 47: Einlassbohrung
- 48: Auslassbohrung
- 49: erstes Ringelement
- 50: zweites Ringelement
- 51: Belüftungslöcher
- 52, 53: Überführungsnut
- 54: Rastnuten
- 55: federndes Rastelement
- 56: Aufhängeinrichtung
- 57: stirnseitige Randfläche
- 58 bis 60: Federzungen
- 61: Handhabe

## Patentansprüche

1. Vorrichtung zur Verabreichung enteraler Ernährung umfassend ein erstes Reservoir (11) sowie ein zweites Reservoir (12), die jeweils über eine Absperrverteilervorrichtung (13) mit einem zum Patienten führenden Anschluss (26) in Verbindung stehen, wobei das erste Reservoir (11) zur Befüllung mit enteraler Ernährung und das zweite Reservoir (12) zur Befüllung mit Flüssigkeit, insbesondere Tee oder Wasser vorgesehen ist,
wobei die Absperrverteilervorrichtung (13) mit mehreren Funktionsstellungen ausgebildet ist, die zumindest ein Herstellen und Unterbrechen einer Strömungsverbindung zwischen dem ersten Reservoir (11) und/oder dem zweiten Reservoir (12) und dem Anschluss (26) umfassen,
**dadurch gekennzeichnet,**
**dass** erstes Reservoir (11), zweites Reservoir (12) und Absperrverteilervorrichtung (13) als zumindest im Betriebszustand miteinander starr verbundene Einheit ausgebildet sind und, dass die Absperrverteilervorrichtung (13) Mittel zur Dosierung des an den Anschluss (26) überführten Fluidstroms umfasst.

2. Vorrichtung nach Anspruch 1,
**dadurch gekennzeichnet,**
**dass** erstes Reservoir (11) und/oder zweites Reservoir (12) und Absperrverteilervorrichtung (13) einstückig zusammenhängend ausgebildet sind.

3. Vorrichtung nach Anspruch 1,
**dadurch gekennzeichnet,**
**dass** erstes Reservoir (11) und/oder zweites Reservoir (12) von der Absperrverteilervorrichtung (13), beispielsweise über einen Schraubanschluss oder über einen Einschnappanschluss (41), lösbar ausgebildet sind.

4. Vorrichtung nach einem der Ansprüche 1 bis 3,
**dadurch gekennzeichnet,**
**dass** erstes Reservoir (11) und zweites Reservoir (12) durch ein gemeinsames Flächenstück, das eine Trennwand (14) zwischen erstem Reservoir (11) und zweitem Reservoir (12) ausbildet, getrennt sind.

5. Vorrichtung nach einem der Ansprüche 1 bis 4,
**dadurch gekennzeichnet,**
**dass** erstes Reservoir (11) und zweites Reservoir (12) ein zusammenhängendes Behältnis (25) mit einer gemeinsamen Außenwand (19) ausbilden.

6. Vorrichtung nach einem der Ansprüche 1 bis 3,
**dadurch gekennzeichnet,**
**dass** das erstes Reservoir (11) und zweites Reservoir (12) jeweils als separate Einheiten ausgebildet und vorzugsweise aneinander über Verkopplungsmittel (28, 29) befestigbar bzw. voneinander lösbar sind.

7. Vorrichtung nach Anspruch 1,
**dadurch gekennzeichnet,**
**dass** die Absperrverteilervorrichtung (13) dazu ausgebildet ist, ein gewünschtes Mischungsverhältnis zwischen Entnahme von enteraler Ernährung aus dem ersten Reservoir (11) und Entnahme von Flüssigkeit aus dem zweiten Reservoir (12) einzustellen.

8. Vorrichtung nach einem der Ansprüche 1 bis 7 ,
**dadurch gekennzeichnet,**
**dass** die Absperrverteilervorrichtung (13) zwei dem ersten Reservoir (11) und dem zweiten Reservoir (12) jeweils zugeordnete, getrennte Dosierventile (30, 31) umfasst.

9. Vorrichtung nach einem der Ansprüche 1 bis 8 ,
**dadurch gekennzeichnet,**
**dass** die Absperrverteilervorrichtung (13) ein gemeinsames dem ersten Reservoir (11) und dem zweiten Reservoir (12) zugeordnetes Misch-/Dosierventil (32) umfasst.

10. Vorrichtung nach einem der Ansprüche 8 bis 9 ,
**dadurch gekennzeichnet,**
**dass** die Absperrverteilervorrichtung (13) ein mit dem ersten Reservoir (11) und/oder dem zweiten Reservoir (12) einstückig verbundenes oder starr verbindbares Außenelement (20) mit mindestens zwei Einlässen und mindestens einem Auslass umfasst, in dem die zwei Dosierventile (30, 31) oder alternativ das Misch-/Dosierventil (32) ausgebildet sind.

11. Vorrichtung nach Anspruch 10 ,
**dadurch gekennzeichnet,**
**dass** die Dosierventile (30, 31) jeweils ein gegenüber dem Außenelement (20) drehbewegliches Innenelement (24) umfassen.

12. Vorrichtung nach Anspruch 10 ,
**dadurch gekennzeichnet,**
**dass** das Misch-/Dosierventil (32) ein gegenüber dem Außenelement (20) sowohl drehbeweglich als auch translatorisch verschiebbares Innenelement (24') umfasst.

13. Vorrichtung nach einem der Ansprüche 11 bis 12 ,
**dadurch gekennzeichnet,**
**dass** das Innenelement (24) der Dosierventile (30, 31) oder das Innenelement (24') des Misch-/Dosierventils (32) mindestens eine um einen vorgegebenen Winkelbereich, vorzugsweise von etwa 140°, umlaufende, in ihrer Tiefe stetig zunehmende Dosiernut (33) aufweist.

14. Vorrichtung nach Anspruch 13 ,
**dadurch gekennzeichnet,**
**dass** die Dosiernut (33) im Innenelement (24) der Dosierventile (30, 31) mit einer parallel zur Dosiernut (33) verlaufenden Sammelnut (34) in Fluidverbindung steht, mittels der das Fluid an eine Mischstelle (36) übergeben und ggf. nach Vermischung mit der jeweils anderen Substanz an den Anschluss (26) geführt wird.

15. Vorrichtung nach Anspruch 12,
**dadurch gekennzeichnet,**
**dass** das Innenelement (24') des Misch-/Dosierventils (32) zwei parallel zueinander verlaufende, in der Zunahme Ihrer Tiefe jedoch entgegengesetzt ausgerichtete Dosiernuten (34, 35), die jeweils einem der beiden Reservoire (11, 12) zugeordnet sind, aufweist.

16. Vorrichtung nach Anspruch 15 ,
**dadurch gekennzeichnet,**
**dass** zwischen beiden Dosiernuten (34, 35) eine Mischnut (37) angeordnet ist und beide Dosiernuten (34, 35) mit der Mischnut (37) in Fluidverbindung stehen, wobei in der Mischnut (37) eine Vermischung der aus den beiden Dosiernuten (34, 35) austretenden Fluidströme stattfindet.

17. Vorrichtung nach einem der Ansprüche 12 bis 16,
**dadurch gekennzeichnet,**
**dass** das Innenelement (24') des Misch-/Dosierventils (32) an seinem Außenumfang einen Abschnitt aufweist, der einen Dosierkonus (38) ausbildet, um durch translatorische Verschiebung des Innenelements (24') relativ zum Außenelement (20) eine Dosierung des an den Anschluss (26) überführten Fluidstroms zu ermöglichen.

18. Vorrichtung nach Anspruch 17 ,
**dadurch gekennzeichnet,**
**dass** zwischen Mischnut (37) und Dosierkonus (38) eine Überführungsnut (42) ausgebildet ist, um Fluid aus der Mischnut (37) an den Dosierkonus (38) zu überführen.

19. Vorrichtung nach einem der Ansprüche 11 bis 18 ,
**dadurch gekennzeichnet,**
**dass** das Innenelement (24) der Dosierventile (30, 31) oder das Innenelement (24') des Misch-/Dosierventils (32) Rastnasen (39) aufweist, um das Innenelement (24, 24') in zugeordneten Rücksprüngen (40) im Außenelement (20) zu verrasten.

20. Vorrichtung nach einem der Ansprüche 1 bis 19 ,
**dadurch gekennzeichnet,**
**dass** erstes Reservoir (11) und zweites Reservoir (12) jeweils mit einem Fassungsvolumen von 600 ml bis 1.500 ml, bevorzugt von jeweils 1.000 ml ausgebildet sind.

21. Vorrichtung nach einem der Ansprüche 1 bis 6 ,
**dadurch gekennzeichnet,**
**dass** erstes Reservoir (11) und/oder zweites Reservoir (12) an ihrer der Absperrverteilervorrichtung (13) abgewandten Seite jeweils eine, vorzugsweise mit Deckel (17, 18) verschließbare, Befüllöffnung (15, 16) zur Nachfüllung von enteraler Ernährung bzw. Flüssigkeit umfassen.

22. Vorrichtung nach Anspruch 21,
**dadurch gekennzeichnet,**
**dass** die Befüllöffnung (15, 16) zumindest im Wesentlichen dem Innenquerschnitt des zugeordneten Reservoirs (11, 12) entspricht.

23. Vorrichtung nach einem der Ansprüche 11 bis 22,
**dadurch gekennzeichnet,**
**dass** Außenelement (20) und Innenelement (24) so ausgebildet und aufeinander abgestimmt sind, dass für das Einstellen des Mischungsverhältnisses zwischen Entnahme aus dem ersten Reservoir (11) und Entnahme aus dem zweiten Reservoir (12) und/oder für das Einstellen des Fluidstroms/der Abgabemenge an den Anschluss (26) die Absperrverteilervorrichtung (13) eine vorbestimmte Anzahl diskreter Rastpositionen umfasst, um das Mischungsverhältnis bzw. die Abgabemenge in diskreten Schritten einstellen zu können.

24. Vorrichtung nach einem der Ansprüche 11 bis 22,
**dadurch gekennzeichnet,**
**dass** Außenelement (20) und Innenelement (24) so ausgebildet und aufeinander abgestimmt sind, dass eine kontinuierliche Einstellung des Mischungsverhältnisses zwischen Entnahme aus dem ersten Reservoir (11) und Entnahme aus dem zweiten Reservoir (12) und/oder für das Einstellen des Fluidstroms/der Abgabemenge an den Anschluss (26) vorgesehen ist.

## Claims

1. A device for administration of enteral nutrition, comprising a first reservoir (11) as well as a second reservoir (12) each communicating, through a shut-off distributor device (13), with a connection (26) leading to the patient, the first reservoir (11) being provided for being filled with enteral nutrition, and the second reservoir (12) being provided for being filled with liquid, in particular tea or water,
the shut-off distributor device (13) being configured so as to have several functional positions including at least an establishment and shut-off of a flow connection between the first reservoir (11) and/or the second reservoir (12) and the connection (26),
**characterized in that**
the first reservoir (11), the second reservoir (12) and the shut-off distributor device (13) are configured so as to be a rigidly interconnected unit at least in the operating state, and that the shut-off distributor device (13) comprises means for metering the fluid flow transferred to the connection (26).

2. The device according to claim 1,
**characterized in that**
the first reservoir (11) and/or the second reservoir (12) and the shut-off distributor device (13) are configured so as to be continuous as one piece.

3. The device according to claim 1,
**characterized in that**
the first reservoir (11) and/or the second reservoir (12) is/are configured so as to be releasable from the shut-off distributor device (13), for example through a screw connection or a snap-in connection (41).

4. The device according to any one of claims 1 through 3,
**characterized in that**
the first reservoir (11) and the second reservoir (12) are separated by a shared patch which forms a partition (14) between the first reservoir (11) and the second reservoir (12).

5. The device according to any one of claims 1 through 4,
**characterized in that**
the first reservoir (11) and the second reservoir (12) form a continuous receptacle (25) with a shared exterior wall (19).

6. The device according to any one of claims 1 through 3,
**characterized in that**
the first reservoir (11) and the second reservoir (12) each are formed as separate units and preferably are connectable to each other or releasable from each other through coupling means (28, 29).

7. The device according to claim 1,
**characterized in that**
the shut-off distributor device (13) is configured for adjusting a desired mixing ratio between a withdrawal of enteral nutrition from the first reservoir (11) and a withdrawal of liquid from the second reservoir (12).

8. The device according to any one of claims 1 through 7,
**characterized in that**
the shut-off distributor device (13) comprises two separate metering valves (30, 31), associated to the first reservoir (11) and the second reservoir (12), respectively.

9. The device according to any one of claims 1 through 8,
**characterized in that**
the shut-off distributor device (13) includes a shared mixing/metering valve (32) associated to the first reservoir (11) and the second reservoir (12).

10. The device according to any one of claims 8 through 9,
**characterized in that**
the shut-off distributor device (13) includes an outer element (20) having at least two inlets and at least one outlet, and connected integrally or rigidly connectable to the first reservoir (11) and/or the second reservoir (12), in which outer element (20) the two metering valves (30, 31) or, alternatively, the mixing/metering valve (32) is/are formed.

11. The device according to claim 10,
**characterized in that**
the metering valves (30, 31) each include an inner element (24) rotatable with respect to the outer element (20).

12. The device according to claim 10,
**characterized in that**
the mixing/metering valve (32) includes an inner element (24') rotatable as well as translationally displaceable with respect to the outer element (20).

13. The device according to any one of claims 11 through 12,
**characterized in that**
the inner element (24) of the metering valves (30, 31) or the inner element (24') of the mixing/metering valve (32) comprises at least one metering groove (33) continuously increasing in its depth, and extending around at a predefined angular range, preferably of about 140°.

14. The device according to claim 13,
**characterized in that**
the metering groove (33) in the inner element (24) of the metering valves (30, 31) is in fluid communication with a collecting groove (34) extending in parallel to the metering groove (33), by means of which the fluid is delivered to a mixing point (36) and is guided to the connection (26), if applicable after mixing with the respective other substance.

15. The device according to claim 12,
**characterized in that**
the inner element (24') of the mixing/metering valve (32) comprises two metering grooves (34, 35) extending in parallel to each other, but oriented in opposite directions in the increase of their depth, which are each assigned to one of the two reservoirs (11, 12).

16. The device according to claim 15,
**characterized in that**
between the two metering grooves (34, 35) a mixing groove (37) is arranged, and both of the metering grooves (34, 35) are in fluid communication with the mixing groove (37), a mixing of the fluid flows exiting from the two metering grooves (34, 35) taking place within the mixing groove (37).

17. The device according to any one of claims 12 through 16,
**characterized in that**
the inner element (24') of the mixing/metering valve (32) comprises a portion at its outer circumference, which forms a metering cone (38), so as to enable a metering of the fluid flow transferred to the connection (26) by a translational displacement of the inner element (24') relative to the outer element (20).

18. The device according to claim 17,
**characterized in that**
a transfer groove (42) is formed between the mixing groove (37) and the metering cone (38) for transferring fluid from the mixing groove (37) to the metering cone (38).

19. The device according to any one of claims 11 through 18,
**characterized in that**
the inner element (24) of the metering valves (30, 31) or the inner element (24') of the mixing/metering valve (32) comprises locking noses (39) for locking the inner element (24, 24') within associated recesses (40) in the outer element (20).

20. The device according to any one of claims 1 through 19,
**characterized in that**
the first reservoir (11) and the second reservoir (12) each are formed so as to have a volumetric capacity of between 600 ml and 1,500 ml, preferably of 1,000 ml each.

21. The device according to any one of claims 1 through 6,
**characterized in that**
the first reservoir (11) and/or the second reservoir (12), at the side thereof facing away from the shut-off distributor device (13), each comprise a filling opening (15, 16) preferably sealable by a cover (17, 18), for replenishing enteral nutrition or liquid.

22. The device according to claim 21,
**characterized in that**
the filling opening (15, 16) at least essentially corresponds to the inner cross section of the associated reservoir (11, 12).

23. The device according to any one of claims 11 through 22,
**characterized in that**
the outer element (20) and the inner element (24) are configured and adapted to each other such that for the adjustment of the mixing ratio between a withdrawal from the first reservoir (11) and a withdrawal from the second reservoir (12) and/or for the adjustment of the fluid flow/the delivery amount to the connection (26), the shut-off distributor device (13) includes a predetermined number of discrete locking positions for being able to adjust the mixing ratio or the delivery amount in discrete steps.

24. The device according to any one of claims 11 through 22,
**characterized in that**
the outer element (20) and the inner element (24) are configured and adapted to each other such that a continuous adjustment of the mixing ratio is provided between a withdrawal from the first reservoir (11) and a withdrawal from the second reservoir (12) and/or for the adjustment of the fluid flow/the delivery amount to the connection (26).

## Revendications

1. Dispositif d'administration d'alimentation entérale comprenant un premier réservoir (11) ainsi qu'un second réservoir (12) qui sont, chacun via un dispositif d'arrêt et de distribution (13), en liaison avec un raccord (26) menant au patient, le premier réservoir (11) étant prévu pour être rempli d'alimentation entérale et le second réservoir (12) pour être rempli de liquide, en particulier de thé ou d'eau, le dispositif d'arrêt et de distribution (13) étant constitué avec plusieurs positions fonctionnelles qui comprennent au moins un établissement et une interruption d'une liaison d'écoulement entre le premier réservoir (11) et/ou le second réservoir (12) et le raccord (26),
**caractérisé en ce que**
le premier réservoir (11), le second réservoir (12) et le dispositif d'arrêt et de distribution (13) sont constitués de façon à former entre eux, du moins en état de fonctionnement, une unité rigide, et **en ce que** le dispositif d'arrêt et de distribution (13) comprend des moyens pour doser le flux de fluide qui est transféré au raccord (26).

2. Dispositif selon la revendication 1,
**caractérisé en ce que**
le premier réservoir (11) et/ou le second réservoir (12) et le dispositif d'arrêt et de distribution (13) sont constitués en un ensemble cohérent comme une seule pièce.

3. Dispositif selon la revendication 1,
**caractérisé en ce que**
le premier réservoir (11) et/ou le second réservoir (12) sont constitués de façon à pouvoir être détachés du dispositif d'arrêt et de distribution (13), par exemple au moyen d'un raccord à vis ou d'un raccord à clipper (41).

4. Dispositif selon l'une des revendications 1 à 3,
**caractérisé en ce que**
le premier réservoir (11) et le second réservoir (12) sont séparés par un élément de surface commun qui constitue une paroi de séparation (14) entre le premier réservoir (11) et le second réservoir (12).

5. Dispositif selon l'une des revendications 1 à 4,
**caractérisé en ce que**
le premier réservoir (11) et le second réservoir (12) constituent un récipient (25) cohérent ayant une paroi extérieure commune (19).

6. Dispositif selon l'une des revendications 1 à 3,
**caractérisé en ce que**
le premier réservoir (11) et le second réservoir (12) constituent chacun une unité distincte, et sont de préférence attachables l'un à l'autre ou détachables l'un de l'autre via des moyens de couplage (28, 29).

7. Dispositif selon la revendication 1,
**caractérisé en ce que**
le dispositif d'arrêt et de distribution (13) est constitué pour régler un rapport de mélange souhaité entre le soutirage d'alimentation entérale depuis le premier réservoir (11) et le soutirage de liquide depuis le second réservoir (12).

8. Dispositif selon l'une des revendications 1 à 7,
**caractérisé en ce que**
le dispositif d'arrêt et de distribution (13) comprend deux vannes doseuses (30, 31) distinctes, associées l'une au premier réservoir (11), l'autre au second réservoir (12).

9. Dispositif selon l'une des revendications 1 à 8,
**caractérisé en ce que**
le dispositif d'arrêt et de distribution (13) comprend une vanne mélangeuse-doseuse (32) commune associée au premier réservoir (11) et au second réservoir (12).

10. Dispositif selon l'une des revendications 8 à 9,
**caractérisé en ce que**
le dispositif d'arrêt et de distribution (13) comprend un élément extérieur (20) relié d'une pièce ou pouvant être relié de manière rigide au premier réservoir (11) et/ou au second réservoir (12), comprenant au moins deux entrées et au moins une sortie et dans lequel sont constituées les deux vannes doseuses (30, 31) ou en variante la vanne mélangeuse-doseuse (32).

11. Dispositif selon la revendication 10,
**caractérisé en ce que**
les vannes doseuses (30, 31) comprennent chacune un élément intérieur (24) mobile en rotation par rapport à l'élément extérieur (20).

12. Dispositif selon la revendication 10,
**caractérisé en ce que**
la vanne mélangeuse-doseuse (32) comprend un élément intérieur (24') mobile à la fois en rotation et en translation par rapport à l'élément extérieur (20).

13. Dispositif selon l'une des revendications 11 à 12,
**caractérisé en ce que**
l'élément intérieur (24) des vannes doseuses (30, 31) ou l'élément intérieur (24') de la vanne mélangeuse-doseuse (32) présente une rainure de dosage (33) s'enveloppant et augmentant constamment en sa profondeur selon une plage angulaire prédéterminée, de préférence de l'ordre de 140°.

14. Dispositif selon la revendication 13,
**caractérisé en ce que**
la rainure de dosage (33), dans l'élément intérieur (24) des vannes doseuses (30, 31), est en liaison fluidique avec une rainure collectrice (34) parallèle à la rainure de dosage (33) et au moyen de laquelle le fluide est remis à un point de mélange (36) et mené au raccord (26) après, le cas échéant, mélange avec l'autre substance.

15. Dispositif selon la revendication 12,
**caractérisé en ce que**
l'élément intérieur (24') de la vanne mélangeuse-doseuse (32) présente deux rainures de dosage (34, 35) parallèles entre elles mais disposées en sens inverse quant à l'accroissement de leur profondeur, et associées chacune à l'un des deux réservoirs (11, 12).

16. Dispositif selon la revendication 15,
**caractérisé en ce que**
une rainure de mélange (37) est disposée entre les deux rainures de dosage (34, 35) et les deux rainures de dosage (34, 35) sont en liaison fluidique avec la rainure de mélange (37), un mélange des flux de fluide qui sortent des deux rainures de dosage (34, 35) s'effectuant dans la rainure de mélange (37).

17. Dispositif selon l'une des revendications 12 à 16,
**caractérisé en ce que**
l'élément intérieur (24') de la vanne mélangeuse-doseuse (32) présente sur sa périphérie extérieure une portion qui constitue un cône doseur (38) pour permettre, par translation de l'élément intérieur (24') par rapport à l'élément extérieur (20), un dosage du flux de fluide qui est transféré au raccord (26).

18. Dispositif selon la revendication 17,
**caractérisé en ce que**
une rainure de transfert (42) est constituée entre la rainure de mélange (37) et le cône doseur (38) pour transférer du fluide de la rainure de mélange (37) au cône doseur (38).

19. Dispositif selon l'une des revendications 11 à 18,
**caractérisé en ce que**
l'élément intérieur (24) des vannes doseuses (30, 31) ou l'élément intérieur (24') de la vanne mélangeuse-doseuse (32) présente des taquets d'encliquetage (39) pour encliqueter l'élément intérieur (24, 24') dans l'élément extérieur (20) par retraits associés (40).

20. Dispositif selon l'une des revendications 1 à 19,
**caractérisé en ce que**
le premier réservoir (11) et le second réservoir (12) sont constitués avec une contenance de 600 ml à 1 500 ml chacun, et de préférence de 1 000 ml chacun.

21. Dispositif selon l'une des revendications 1 à 6,
**caractérisé en ce que**
le premier réservoir (11) et/ou le second réservoir (12) comprennent chacun, sur leur côté opposé au dispositif d'arrêt et de distribution (13), une ouverture de remplissage (15, 16) fermable, de préférence par couvercle (17, 18), permettant de faire l'appoint d'alimentation entérale et/ou de liquide.

22. Dispositif selon la revendication 21,
**caractérisé en ce que**
l'ouverture de remplissage (15, 16) équivaut au moins sensiblement à la section transversale intérieure du réservoir associé (11, 12).

23. Dispositif selon l'une des revendications 11 à 22,
**caractérisé en ce que**
l'élément extérieur (20) et l'élément intérieur (24) sont constitués et ajustés l'un par rapport à l'autre de telle façon que pour le réglage du rapport de mélange entre le soutirage depuis le premier réservoir (11) et le soutirage depuis le second réservoir (12) et/ou pour le réglage du flux de fluide / de la quantité à délivrer au raccord (26), le dispositif d'arrêt et de distribution (13) comprend un nombre prédéterminé de positions d'encliquetage discrètes pour pouvoir régler le rapport de mélange ou la quantité à délivrer par pas discrets.

24. Dispositif selon l'une des revendications 11 à 22,
**caractérisé en ce que**
l'élément extérieur (20) et l'élément intérieur (24) sont constitués et ajustés l'un par rapport à l'autre de telle façon qu'un réglage continu du rapport de mélange entre le soutirage depuis le premier réservoir (11) et le soutirage depuis le second réservoir (12) et/ou pour le réglage du flux de fluide / de la quantité à délivrer au raccord (26) est prévu.
